# EUROPEAN PATENT APPLICATION

(11) **EP 1 876 176 A1**
(43) Date of publication of application: **09.01.2008**
(21) Application number: 06743488.6
(22) Date of filing: 06.04.2006
(51) Int. Cl.: C07D 301/04, C07D 301/10, B01J 23/52, B01J 21/06, B01J 29/74, B01J 23/89

(54) **METHOD AND CATALYSTS FOR THE EPOXIDATION OF OLEFINIC COMPOUNDS IN THE PRESENCE OF OXYGEN**

(30) Priority: 19.04.2005 ES 200500994
(71) Applicant: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: CORMA CANOS, Avelino Insto. de Tecnología Química, E-46022 Valencia (ES); DOMINE, Marcelo E., Insto. de Tecnología Química, E-46022 Valencia (ES)
(74) Representative: Pons Arino, Angel
(86) International application number: PCT/ES2006/070044
(87) International publication number: WO 2006/111600

(57) **Abstract**

The invention relates to a method for the epoxidation of an olefinic compound , which is **characterized in that** it comprises an oxidation reaction of at least one olefinic compound containing one or more double bonds C=C with oxygen in the presence of one or more reaction initiating agents, at least one catalyst containing at least one metal that is selected from a noble metal, a transition metal and mixtures of same , and al least one hydrocarbon that is selected from one or more branched alkanes, one or more alkyl cyclic or cyclic hydrocarbons, one or more alkyl aromatic or aromatic compounds and mixtures of same.

## Description

### TECHNICAL FIELD OF THE INVENTION

Oxidation, Heterogeneous Catalysis, Petrochemicals

### BACKGROUND

For many years now, the epoxidation of olefinic compounds has attracted considerable interest on the part of researchers because of the versatility of epoxides as intermediates in organic synthesis [K. A. Jorgensen, Chem. Rev., 89, 431, 1989]. Of particular interest in industrial terms are oxygenated derivatives of olefins of low molecular weight (ethylene, propylene, butylene) for obtaining glycols, polyglycols and polyethers, widespread use of which is made in the detergent, polymer, resin, varnish and paint industries, among many others [*"*Kirk-Othmer Encyclopedia of Chemical Technology", Vol. 9, 4th Ed., J. Wiley & Sons, NY, 1994, p. 915].

The main commercial routes for obtaining low molecular weight epoxides (e.g.: propylene oxide, PO) are processes which employ chlorohydrin and hydroperoxides, both in liquid state. The chlorohydrin process causes serious problems of corrosion in the reactors and environmental pollution, and is also carried out under highly hazardous conditions, leading to its gradual replacement by the use of hydroperoxides or other alternative systems of epoxidation. There are various alternative processes for the production of low molecular weight epoxides using hydroperoxides. For example, the Halcon-ARCO process [J. P. Schmidt (Oxirane Corp.), US Pat 3,988,353 , 1976] uses a Mo catalyst in homogeneous state, and as oxidant ethylbenzene hydroperoxide. The conversion rates of propylene achieved are 84% after 75 minutes, with the drawback that a number of steps are involved in the final separation of the catalyst from the reaction mixture. In order to avoid the stages of separation, the company Shell Oil developed a heteregeneous silica-titania catalyst, particularly active in the epoxidation of olefins with ethylbenzene hydroperoxide [H. P. Wulff et al. (Shell Oil Co.), GB Pat 1,249,079, 1971]. For more than a decade, many research projects undertaken by university and corporate groups have devised a number of different methods for preparing silica-titania materials, improving the efficiency of these catalysts in the olefin epoxidation process [A. Baiker et al., J. Catal., 153, 177, 1995; and Catal. Rev.Sci. Eng., 42(1-2), 213, 2000].

Meanwhile, ENICHEM's technology, based on the use of a solid titano-silicate catalyst, TS-1, and hydrogen peroxide as the oxidant in liquid state, provides high conversion and selectivity rates for the epoxides [M. Taramaso et al. (SNAM Progetti), US Pat 4,410,501, 1983; M. G. Clerici et al. (ENICHEM Sintesi S.p.A.), US Pat 4,824,976, 1989]. The results are even better if the titanium silicate (TS-1) is modified at a stage following the synthesis, achieving epoxide (PO) selectivity rates of 97%, with 90% conversion rates for the oxidant [M. G. Clerici et al. J. Catal., 129, 159, 1991].

The above catalysts have been combined with new micro- and meso-porous molecular sieves containing Ti. For example, while micro-porous materials such as Ti-Beta [A. Corma et al., J. Chem. Soc, Chem. Commun., 589, 1992; and J. Catal., 145, 151, 1994] are capable of efficiently epoxidising olefins using H₂O₂, mesoporous materials of the type Ti-MCM- 41 [A. Corma et al., WO 9429022 A1, 1994] and Ti-MCM-48 [A. Corma et al., WO 0 04460 A1, 2000], among others, allow a wide range of olefinic molecules to be oxidised using organic hydroperoxides as oxidants [A. Corma et al., WO-2000054880 A1, 2000].

Other methods have been put forward as alternatives to those already in existence for the synthesis of olefin epoxides. One of these involves the epoxidation reaction of olefins in gaseous state with H₂O₂ generated in situ from H₂ and O₂ on solid catalysts containing various noble metals. For example, catalysts of the type Pd-Pt/TS-1 have proved to be active and selective in the epoxidation of propylene in gaseous state [M. Clerici et al., Catal. Today, 41, 14, 1998; R. Meiers et al., Catal. Lett., 59,161, 1999; and J. Catal., 176, 376, 1998], although the epoxide yield achieved is close to 5%.

In addition, the doping of silica-titania type materials, in addition to various transition metal oxides using noble metal particles (for example, Au, Pd, Pt) has made possible to generate H₂O₂ within the reaction medium itself using H₂ and O₂, consequently obtaining the epoxide in a clean and efficient manner [M. Haruta and cowork., Stud. Surf. Sci. Catal., 110, 965, 1997; and J. Catal., 186(1), 228, 1999]. Of all these, materials based on Au and Titanium give the best results, with epoxide selectivity rates = 99%, although in no case are the olefin conversion rates obtained greater than 2%, making the strong deactivation of the catalyst the major drawback [J. A. Moulijn and cowork., Catal. Today, 72,59, 2002], in addition to the need to work under conditions involving H₂ and O₂ mixtures close to the explosion threshold in order to achieve greater yields.

An ideal process for obtaining epoxides cleanly and efficiently would involve the direct and selective oxidation of the olefin with molecular oxygen in gaseous state. While the direct oxidation of ethylene with O₂ has been known of since the end of the 19th century, it was not until 1930 that an active and selective silver catalyst was used for the process [T. E. Lefort, 175-1,998,878, 1935], and this continues to dominate the market for the production of ethylene oxide. However, and despite the efforts made in the epoxidation of other low molecular weight mono-olefins with O₂ (e.g. PO), the epoxide yields are generally very poor [M. Clerici et al., J. Catal., 129, 159, 1991]. In addition to the above catalysts, other catalysts based on: Ag supported on mixed oxides [J. Q. Lu et al., Appl. Catal. A:Gral., 237(1-2), 11, 2002], modified molecular sieves containing Ti [K. Murata et al., Chem. Commun., 1356, 2001] and Cu supported on mixed oxides [ J. Lu et al., J. Catal., 211, 552, 2002], have been examined in the direct epoxidation of olefins with O₂, with very poor results.

Other types of catalyst have also been published for this process, such as: Au-Ti/SiO₂ [T. Hayashi et al. (Nippon Shokubai Co. , Ltd.), EP Pat 1040860 A2, 2000], Ag-Mo-Na-Cs/Al₂O₃ [H. Borchert et al. (BASF Aktiengesellschft), EP Pat 1393801 A1, 2004], or binary combinations of Rh with various transition metals (V, Cr, Sn, In, Mo, Sm) supported on Al2O3 [T. Miyazaki et al., Catal. Today, 81 (3), 473, 2003] . All the above reveal very low yields of the desired epoxide.

In light of all the above, there is a clear need to develop alternative catalytic processes for the production of organic epoxides, by means of oxidation in the presence of 0₂, and which would be economically and industrially profitable viable.

### DESCRIPTION OF THE INVENTION

The present invention refers to a procedure for the epoxidation of olefinic compounds, characterised in that it involves performing an oxidation reaction of a least one olefinic compound containing one or more double C=C bonds, with oxygen, in the presence of:
- one or more reaction initiated agents, and at least one catalyst, comprising at least one metal selected from among one or more noble metals, one or more transition metals and mixtures thereof, and
- at least one hydrocarbon selected from among one or more branched alkanes, one or more cyclic or alkyl-cyclic hydrocarbons, one or more aromatic or alkyl-aromatic compounds and mixtures thereof. In this report the term "olefinic compound" refers to an organic compound with at least one double carbon-carbon (C=C) bond, irrespective of whether it has other functional groups.

In accordance with the implementation of any procedure of the present invention, the organic epoxides obtained are preferably terminal, internal, linear or cyclic olefin epoxides, with no branching, with one or more chain branches, and containing a hydrocarbon chain of between 2 and 24. The organic epoxides which may be obtained may correspond to the general formula:

R₁-HCOCH-R₂

Where R₁ and R₂ are the same or different replacements, selected freely from among: alkyl with from 1 to 12 C atoms, linear or branched, substituted or unsubstituted; cyclic alkyl with from 4 to 12 C atoms, substituted or unsubstituted; or aryl with from 6 to 18 C atoms, substituted or unsubstituted.

In accordance with one implementation of the present invention, the organic epoxides have the formula:

R₁-HCOCH- (CH₂) ₙ-HCOCH-R₂

where R₁ and R₂ are equal or different replacements, selected from among any of: alkyl with from 1 to 12 C atoms, linear or branched, substituted or unsubstituted; cyclic alkyl with from 4 to 12 C atoms, substituted or unsubstituted; or aryl with from 6 to 18 C atoms, substituted or unsubstituted; and n may vary between 1 and 12.

In accordance with one specific implementation of the present invention, the organic epoxides obtained have between 2 and 12 carbon atoms.

Examples of the epoxides would include ethylene oxide, propylene oxide, 1,2-epoxy-butane, 1,2-epoxy-hexane, 1,2-epoxy-octane, 1,2-epoxy-cyclcohexane, 1,2-epoxy-1-methyl-cyclohexane, among others, without being limited to the above examples.

In accordance with the procedure of the present invention, the olefinic compound or compounds selected are organic compounds with one or more double bonds in their structure, preferably mono-, di- or polyolefins. The olefinic compound is preferably a mono-olefin. The said mono-olefin is preferably selected from among one or more terminal olefins, internal olefins, branched olefins, cyclic olefins, and combinations thereof.

The said hydrocarbon may be selected from among compounds corresponding to the formula

R₁-(CH₂)ₙ-R₂

where R₁ and R₂ are equal or different replacements, selected indistinctly from among any of: hydrogen, branched alkyl with from 1 to 12 C atoms, substituted or unsubstituted; cyclic alkyl with from 4 to 12 C atoms, substituted or unsubstituted; or aryl with from 6 to 18 C atoms, substituted or unsubstituted; and n may vary between 0 and 12.

Preferably, the branched alkane or hydrocarbon may be selected from among sec-alkanes, cycloalkanes, alkyl-cycloalkanes, aryl-cycloalkanes, alkyl-aromatics, or mixtures thereof. Examples of these would include: sec-alkanes with 3 or more carbon atoms, such as isobutane, 2-methyl-pentane, 3-methyl-pentane, 2-methyl-hexane, 3-methyl-hexane, 3-methyl-heptane, 4-methyl-heptane; cycloalkanes and substituted cycloalkanes, such as cyclohexane, methyl-cyclohexane, di-methyl-cyclohexane; and alkyl-aromatics, such as ethyl-benzene, iso-propyl-benzene (cumene), di-iso-propyl-benzene, without being limited to the above examples.

In accordance with a preferred implementation of the procedure, the olefinic compound is propylene and the hydrocarbon is a methyl-alkane, such as methyl-pentane, or mixtures of methyl-alkanes.

In accordance with an additional preferred implementation of the procedure, the olefinic compound is propylene, and the hydrocarbon is ethyl-benzene.

In accordance with an additional preferred implementation of the procedure, the olefinic compound is propylene and the hydrocarbon is iso-propyl-benzene (cumene).

The reaction takes place in the presence of one or more activator or initiator agents.

The reaction initiator agent may be selected from among:
- one or more organic nitriles,
- one or more azo-compounds,
- mixtures of one or more organic nitriles and one or more azo-compounds, and
- one or more organic nitriles comprising an azo- group in the same molecule.

The organic nitriles are preferably used in combination with azo-groups in the same molecule.

Preferably, and in the case of using compounds with nitrile-type functional groups, preferably in combination with azo- groups in the same molecule, the compounds used correspond to the general formula:

N≡C-R₁-N=N-R₂-C≡N

where R₁ and R₂ are the same or different replacements, selected freely from among: an alkyl group of from 1 to 12 C atoms, linear or branched, substituted or unsubstituted; an alkyl-cyclic group of from 4 to 12 C atoms, substituted or unsubstituted; an aryl group from 6 to 18 C atoms, substituted or unsubstituted. The organic nitrile preferably has an alkyl-azo group of from 1 to 12 C atoms, linear or branched, substituted or unsubstituted; a bis-alkyl-azo group of from 1 to 12 C atoms, linear or branched, substituted or unsubstituted; an aryl-azo group of from 6 to 18 C atoms, substituted or unsubstituted; a bis-aryl-azo group of from 6 to 18 C atoms, substituted or unsubstituted.

Unrestrictive examples of the organic nitriles employed could include: aceto-nitrile, butyro-nitrile, iso-butyro-nitrile, phenyl-nitrile, and more preferably, azo-butyro-nitrile, azo-iso-butyro-nitrile, azo-bis-iso-butyro-nitrile, azo-phenyl-nitrile and azo-bis-phenyl-nitrile.

In accordance with the procedure of the present invention, the oxygen may be derived from a source selected from among molecular oxygen in pure form, a gaseous mixture comprising oxygen and combinations of the two.

The said gaseous mixture comprising oxygen may be selected from among air, oxygen-enriched air, oxygen-enriched ozone, oxygen-enriched N₂, oxygen-enriched Ar and a mixture comprising two or more gases, for example a mixture of nitrogen, argon and oxygen, these examples being non-restrictive. The quantity of oxygen and the source selected will depend on the type of reactor and the specific reaction conditions of the process. The quantity of oxygen present in the reactive medium will always be in accordance with the initial quantity of the olefinic compound employed, and will depend on the reactor temperature and pressure, in order to minimise any undesired secondary reactions.

The catalyst in accordance with the procedure of the present invention may be selected from among:
a) a metallic catalyst *"CAT A"* comprising:
   - one or more noble metals, or
   - one or more transition metals, or
   - one or more of their salts or complexes, and
   - combinations of the above,
   with the aforementioned *"CAT A"* being supported on, or included within, the structure of an inorganic matrix;
b) a metallic catalyst "CAT T" comprising one or more transition metals, their salts or complexes, included within or supported on the structure of an inorganic matrix; and c) combinations thereof: *"CAT A"* + *"CAT T".*

In the catalysts used in the procedure described, the metal or metals may be in the form of salts or complexes, such as noble metal complexes or transition metal complexes. When the catalyst is a metallic catalyst "CAT A" , it may include one or more noble metals, one or more transition metals, or one of their salts, and be supported on, or included within, the structure of an inorganic matrix. The said noble metal may be selected from, for example, among Au, Pd, Pt, Ag, Re, Rh, and combinations thereof. Preferably, the said noble metal is Au or Au combined with another metal. More preferably, even if the catalyst is *"CAT A",* the said Au, or Au combined with another metal, is supported in the form of nano-particles of a size of between 0.5 and 20 nm.

In any implementation of the procedure, the catalyst may be "CAT *A", "CAT T" or CAT A* + *CAT T,* with the said transition metal being selected from among one or more metals of the groups Ib, IIb, IVb, Vb, VIb, VIIb and VIII of the periodic table. In accordance with preferred implementations, the said transition metal is selected from among Ti, Zr, Zn, Cu, Co, Mn, Mo, V, Ni, Fe, Al, and combinations thereof.

When the catalyst used in the procedure is *"CAT A",* the said catalyst may be supported on, or included within, the structure of an inorganic matrix which may be an amorphous material selected from among one or more metal oxides, one or more mixed metal oxides, and combinations thereof. The said amorphous materials may include silica, alumina, ceria, yttria, titania, Fe₂O₃, silica-alumina, silica-ceria, one or more mixed alkaline earth metal oxides, and one or more transition metal oxides. Preferably, the said inorganic matrix is a cerium oxide.

When the catalyst is "CAT A", the said solid or inorganic matrix may also be of the type of micro-porous molecular sieves, meso-porous molecular sieves, and combinations thereof.

In another specific case, the metallic catalyst "CAT A" may comprise a compound selected from among at least one salt and one transition metal complex, the said salt or complex supported on, or included within, the structure of a solid or inorganic matrix, such as amorphous solids, or of the type of micro-porous molecular sieves, meso-porous molecular sieves and combinations thereof.

Non-restrictive examples of the amorphous solid matrices used could also include: silica, alumina, ceria, yttria, titania, oxides of Fe such as Fe₂O₃, silica-alumina, silica-ceria, and in general mixed metal and/or transition metal oxides such as Cu, Co, Zr, Zn, Ti, Mn, V, Ni, Fe, Mo, among others.

Non-restrictive examples of the amorphous solid matrices used could include solids made up of alkaline earth metal oxides (MgO, CaO, BaO), preferably MgO, together with oxides of other types of metal, and in general mixed oxides derived from anionic clays, such as for example double laminar hydroxides of the hydrotalcite type (Mg/Al).

Non-restrictive examples of the micro-porous solid matrices employed could include: micro-porous silicates, comprising pure silica zeolites, micro-porous alumino-silicates, comprising Al-zeolites, micro-porous metal-silicates, comprising Me-zeolites, micro-porous alumino-phosphates (ALPOs, APOs and similar), micro-porous alumino-phosphates containing metals (Me-APOs), micro-porous silico-alumino-phosphates (SAPOs, TAPSOs, etc.). The inorganic matrices employed may also be laminar micro-porous inorganic materials, such as clays and pillared clays, of the bentonite, montmorillonite and other types, or combinations thereof. Non-restrictive examples of the meso-porous solid matrices employed could include: silicates, alumino-silicates, and in general meso-porous metal-silicates with a hexagonal or cubic structure, such as MCM-41, MCM-48, SBA-15, HMS, MSA, among others. The meso-porous solid matrices employed may also comprise meso-porous materials obtained through the delamination of laminar zeolitic precursors, such as ITQ-2, ITQ-6, among others.

Whether the catalyst is "CAT A", "CAT T" or a combination of the two, the said catalyst may be included within an inorganic matrix of one or more micro-porous molecular sieves. The said micro-porous molecular sieve is selected from among a zeolite, clay, pillared clay, and mixtures thereof.

Whether the catalyst is "CAT A", "CAT T" or a combination of the two, the said catalyst may be included within an inorganic matrix of one or more meso-porous molecular sieves, the said meso-porous molecular sieves being potentially selected from among silicate, metal-silicate and a meso-porous material derived from the delamination of a laminar zeolitic precursor.

In accordance with the procedure of the invention and as stated above, the catalyst may be a "CAT T" catalyst, comprising one or more transition metals, included within or supported on the structure of an inorganic matrix. In accordance with specific implementations, the said inorganic matrix is an amorphous material selected from among: silica, alumina, silica-alumina, titania, silica-titania, and a mixed transition metal oxide.

In accordance with additional specific implementations, when the catalyst is "CAT T", the said meso-porous solid is selected from among meso-porous molecular sieves, and molecular sieves containing meso- and micro-pores, and contains at least Si, Ti incorporated within the grid, in tetrahedral positions. It may also occur that in a specific implementation the said meso-porous solid may additionally comprise Ti in non-reticular (octahedral) positions of the molecular sieve, and silicon bonded to carbon.

In another specific case of the epoxidation procedure described in the present invention, the metallic catalyst *"CAT T"* may comprise a micro-porous molecular sieve, a meso-porous molecular sieve, or even amorphous siliceous materials, or those containing Si and Ti, or combinations thereof. Preferably, these materials may contain Si, Ti and Si-C bonds, comprising an organic-inorganic composite.

In accordance with the procedure of the present invention, the said organic-inorganic composite comprising at least Si, Ti and silicon bonded to carbon is obtained by means of a procedure which involves a silylation stage during the synthesis, or alternatively a procedure comprising a post-synthesis silylation stage.

The said organic-inorganic composite may be a micro-porous molecular sieve comprising a least Si, Ti and silicon bonded to carbon, or otherwise a meso-porous molecular sieve comprising at least Si, Ti and silicon bonded to carbon, or may comprise amorphous inorganic siliceous solids chemically combined with Ti in proportions of between 0.2 and 8% by weight of Ti in the form of oxide on the total catalyst, and containing silicon bonded with carbon. The precursor of a meso-porous molecular sieve employed as catalyst may have the chemical formula:

y (Aⁿ⁺_{1/n} XO₂) :tTO₂: (1-m) S, O₂ :xTiO₂ :mR₍₄₋ₚ₎SiOₚ/₂:sS

where
- x has a value of between 0.005 and 0.1, both figures inclusive;
- X represents at least one trivalent element, and has a value of between 0 and 0.2, both figures inclusive;
- A represents mono-, di- or tri-valent compensation cations, or mixtures of these,
- n=1, 2 or 3,
- T represents tetravalent elements other than Si or Ti,
- t lies between 0 and 1, and preferably between 0 and 0.2, both figures inclusive;
- S represents an organic compound,
- s may range from 0 to 0.5, both figures inclusive;
- m lies between 10⁻⁶ and 0.66, both figures inclusive;
- p lies between 3 and 1, both figures inclusive;
- and where R is an alkyl or aromatic group or a combination of the two derived from the silylation agent containing the Si-C bonds.

The organic compound corresponding to the group S is extracted by chemical means and the meso-porous molecular sieve is subjected to a post-synthesis treatment with a silylation agent giving rise to the formation of new Si-C bonds. These materials have an elevated specific surface of between 200 and 1500 m²·g⁻¹ and have an intense band within the UV-Vis spectrum centred around 220 nm, indicating the presence of Ti in tetrahedral settings. The said meso-porous solid materials may include such examples of ordered meso-porous materials as MCM-41, MCM-48, SBH-15, HMS and other amorphous materials, such as amorphous silica. The titanium is introduced in the synthesis stage, or during treatment following synthesis. Additionally, the said materials may present organic groups anchored on their surface.

In accordance with a specific implementation of the procedure, the catalyst may be of the type "CAT A", CAT T", or "CAT A + CAT T", supported on a meso-porous molecular sieve corresponding in its calcined and anhydrous form, without any organic component, to the chemical composition

y *(A*_{*1*/}*ₙ*^{*n*+} X0₂) : t T0₂ : SiO₂ : x TiO₂

where:
- X represents at least one trivalent element,
- y lies between 0 and 0.2, and preferably between 0 and 0.1,
- A represents mono-, di- or tri-valent compensation cations, or mixtures of these, 10 - n = 1, 2 or 3,
- T represents at least one tetravalent element other than Si or Ti,
   t lies between 0 and 1, and preferably between 0 and 0.2, and
- x may range between 0.015 and 0.065; (2 and 8% by weight in the form of TiO₂).

The meso-porous molecular sieve is subjected to a post-synthesis treatment with a silylation agent giving rise to the formation of new Si-C bonds. These materials have an elevated specific surface of between 200 and 1500 m²·g⁻¹ and present an intense band in the UV-Vis spectrum around 220 nm, indicating the presence of Ti in tetrahedral settings.

In accordance with a specific implementation of the procedure, the catalyst may be of the type "CAT A", CAT T", or "CAT A + CAT T", supported on a meso-porous molecular sieve selected from among materials of the type MCM-41, MCM-48, SBA-15, and HMS, and combinations thereof.

The said meso-porous solid may have been prepared by means of a process involving a stage selected from among synthesis and post-synthesis stages, during which Si-C bonds are introduced into the catalyst.

The procedure for the epoxidation of olefinic compounds in the presence of O₂ may be conducted in a discontinuous reactor, a continuous stirred tank reactor (CSTR), a continuous fixed bed reactor, a fluidised bed reactor, or an ebullient bed reactor.

In one implementation of the present invention, the epoxidation procedure of olefinic compounds is undertaken by placing into contact a reactive mixture containing one or more olefinic compounds, a source of oxygen (preferably O₂ or air), an initiator or activator agent, one or more hydrocarbons, and a metallic catalyst *"CAT A",* or a solid material containing metallic forms *"CAT T"*, or a mixture of the two, *"CAT A"* + *"CAT T"*, within a range of pressures which may vary from atmospheric pressure up to 50 bars, at a temperature of between 10 and 250°C, for reaction times which may range from between 2 minutes and 72 hours, depending on the catalyst and the reaction conditions employed.

In the procedure according to the present invention, the weight ratio of the olefinic compound to the catalyst preferably lies within the range between 2 and 1000, and more preferably between 10 and 500.

The weight ratio of the olefinic compound and the oxidation agent may preferably lie within the range between 3 and 600, while the weight ratio of the olefinic compound and the initiator agent lies preferably within the range between 10 and 10000, and the weight ratio of the olefinic compound and the hydrocarbon lies between 0.1 and 200. In the event that the oxidation reaction takes place in a discontinuous reactor, the weight ratio of the olefinic compound and the catalyst is preferably between 2 and 1000, more preferably between 10 and 500. In the case of a discontinuous reactor, the molar ratio between the olefinic compound and the oxygen is between 3 and 600, and oxygen may also be added continuously to the system in controlled quantities to maintain the overall pressure of the reactor constant throughout the process. The temperature of the procedure in a discontinuous reactor preferably lies between 10 and 250°C, more preferably between 40 and 200°C. The reaction time in a discontinuous reactor preferably ranges between 2 minutes and 72 hours. The epoxidation reaction, when it takes place within a discontinuous reactor, is performed at an overall pressure within the system preferably between atmospheric pressure and 50 bars.

When the oxidation reaction takes place in a discontinuous reactor, the weight ratio of the olefinic compound and the initiator agent is preferably within the range between 10 and 10000.

In the event that the oxidation reaction takes place in a discontinuous reactor, the weight ratio of the olefinic compound and the hydrocarbon is preferably between 0.1 and 200.

The present invention describes a process for the direct epoxidation of olefinic compounds, comprising ethylene, propylene, butenes, 1-hexene, 2-hexene, 1-octene, 2-octene, 3-octene, cyclohexene, methyl-cyclohexene, among others, and in general olefinic compounds including in their structure between 2 and 24 carbon atoms, and more specifically between 2 and 12 carbon atoms, with one or more C=C bonds.

By means of the procedure of the present invention, excellent rates may be obtained for conversion and selectivity to the corresponding organic epoxides, both aliphatic and cyclic, through the selective epoxidation of olefinic compounds with O₂ or air, or mixtures of oxygen-enriched gases, using an activator or initiator agent, one or more hydrocarbons, and in the presence of one or more metallic catalysts, under mild or moderate reaction conditions (temperature and pressure).

The following examples illustrate the preparation of the metallic catalysts and their application to the direct and selective epoxidation reaction of olefinic compounds with O₂, in order to obtain organic epoxides.

### EXAMPLES

**Example 1a:** Preparation of a metallic catalyst *"CAT A"* based on a Ce0₂ material containing Au in its composition. [Au/CeO₂]For the deposition of the particles of gold (Au) on the surface of the cerium oxide, the impregnation method was used, with HAuCl4 as the source of Au, following the exerimental procedure detailed below. To 0.6 g of HAuCl4-3H₂O diluted in 70 ml of water (Milli-Q quality) was added (drop by drop) a solution of 0. 2M of NaOH to obtain a pH = 10. This solution of Au salt in water (pH=10) was added to a container holding 5.7 g of Ce0₂ in 200 ml of water (Milli-Q quality), under continuous, vigorous agitation. The mixture so obtained was constantly agitated at room temperature for 15-16 hours, and once the solid had been recovered by filtration, it was washed thoroughly with water and dried using a heater at 100°C for approximately 12 hours. The material thus synthesised was classified adequately using various spectroscopic techniques and chemical methods, finally obtaining a sample of Au/CeO₂ with a solid weight of approximately 2.5% Au.

### Example 1b: Preparation of a metallic catalyst "CAT A" based on a meso-porous molecular sieve of the MCM-41 type containing Au in its composition. [Au/MCM-41]

For the deposition of the particles of gold (Au) on the surface of the porous molecular sieve of the MCM-41 type (molar ratio Si/Al = infinnity), the impregnation method was used, with HAuCl4 as the source of Au, following the exerimental procedure detailed below. To 1.2 g of HAuCl4 • 3H₂O diluted in 100 ml of water (Milli-Q quality) was added (drop by drop) a solution of 0. 2M of NaOH to obtain a pH = 10. This solution of Au salt in water (pH=10) was added to a container holding 5.0 g of the MCM-41 material in 200 ml of water (Milli-Q quality), under continuous, vigorous agitation. The mixture so obtained was constantly agitated at room temperature for 15-16 hours, and once the solid had been recovered by filtration, it was washed thoroughly with water and dried using a heater at 100°C for approximately 12 hours. The material thus synthesised was classified adequately using various spectroscopic techniques and chemical methods, finally obtaining a sample of Au/MCM-41 with a solid weight of approximately 4.5% Au.

### Example 2a: Preparation of a metallic catalyst "CAT T" based on a meso-porous molecular sieve of the MCM-41 type containing Ti in its composition. [Ti-MCM-41]

3.11 g of cetyl trimethyl ammonium bromide (CTAB) are dissolved in 20.88 g of water. To this solution are added 5.39 g of tetramethylammonium hydroxide (TMAOH) and 0.21 g of titanium tetraethoxide (TEOT), and it is agitated until the titanium has fully dissolved. 3.43 g of silica are then added, giving rise to a gel which is agitated at room temperature for 1 hour at 250 rpm. The resulting mixture is placed into autoclaves and heated at 100°C at the system's autogenous pressure for 48 hours. Following this time, a solid is recovered by filtration, washed thoroughly with distilled water and dried at 60°C for 12 hours. The solid material so obtained is placed in a tubular quartz reactor and subjected to a stream of dry nitrogen at a rate of 50 ml·min⁻¹ while the temperature is increased to 540°C at 3°C·min⁻¹. Once this temperature has been reached, nitrogen is passed for 60 minutes, following which the flow of nitrogen is replaced by a flow of dry air at 50 ml·min⁻¹. Calcination is prolonged for a further 360 minutes and the solid is cooled at room temperature. This heat treatment allows all the organic matter occluded in the pores of the material to be completely eliminated. This solid has a specific surface of 950 m2·g⁻¹, and a band in the UV-Vis spectrum centred at 220 nm.

### Example 2b: Preparation of a hybrid metallic organic-inorganic catalyst "CAT T" based on a meso-porous molecular sieve of the MCM-41 type containing Ti in its composition. [Ti-MCM-41-Sil.]

2.0 g of the solid sample obtained in example 2a are dehydrated at 100°C and 10⁻³ Torr for 2 hours. The sample is cooled, and at room temperature a solution of 1.88 g of hexamethyldisilizane (CH3)3Si-NH-Si(CH₃)₃) in 30 g of toluene is added. The resulting mixture is refluxed at 120°C for 90 minutes and washed with toluene. The end product is dried at 60°C. This solid has a specific surface of 935 m2·g⁻¹, and a band in the UV-Vis spectrum centred at 220 nm. Additionally, the spectrum of ²⁹Si-MAS-RMN presents a resonance band at -10 ppm assigned to the presence of Si-C bonds.

### Example 3: Results obtained in the epoxidation of olefinic compounds in the presence of 0₂ with the catalyst described in Example 1 a. [Au/CeO₂ - CAT A]

Into a 12 ml stainless steel autoclave reactor, with an inner lining of Teflon and containing a magnetic stirrer, were placed 3000 mg of 1-octene and 1000 mg of 3-methyl-pentane, followed by the addition of 85 mg of a catalyst as described in Example 1 a [Au/CeO₂- CAT A] . The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector (flame ionisation detector), to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.10 | 58.9 | 10.7 | 30.4 | 0 | 0 |
| 2 | 0.15 | 62.5 | 19.7 | 17.8 | 0 | 0 |
| 3.5 | 0.24 | 73.4 | 13.0 | 13.6 | 0 | 0 |
| 5 | 0.33 | 78.7 | 8.7 | 12.6 | 0 | 0 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of the olefinic compound. | | | | | | |

### Example 4: Results obtained in the epoxidation of olefinic compounds in the presence of O₂ with the catalyst described in Example 2b. [Ti-MCM-41-Sil. - CAT T]

Into a 12 ml stainless steel autoclave reactor, with an inner lining of Teflon and containing a magnetic stirrer, were placed 3000 mg of 1-octene and 1000 mg of 3-methyl-pentane, followed by the addition of 100 mg of a catalyst as described in Example 2b [Ti-MCM-41-Sil. - CAT T] . The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.27 | 95.0 | 1.3 | 3.7 | 0 | 0 |
| 2 | 0.32 | 92.3 | 2.7 | 5.0 | 0 | 0 |
| 3.5 | 0.34 | 80.4 | 5.1 | 7.2 | 7.3 | 0 |
| 5 | 0.35 | 73.4 | 5.4 | 9.5 | 9.3 | 2.4 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

**Example 5:** Results obtained in the epoxidation of olefinic compounds in the presence of O₂ with the combination of catalysts described in Examples 1 a and 2b. [Au/CeO₂ - CAT A] + [Ti-MCM-41-Sil. - CAT T]Into a 12 ml stainless steel autoclave reactor, with an inner lining of Teflon and containing a magnetic stirrer, were placed 3000 mg of 1-octene and 1000 mg of 3-methyl-pentane, followed by the addition of a mechanical mixture of 50 mg of a catalyst as described in Examples 1 a [Au/CeO₂ - CAT A] + 100 mg of a catalyst as described in Example 2b [Ti-MCM-41-Sil. - CAT T] . The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | - | - | - | - | - | - |
| 2 | 0.15 | 62.5 | 19.7 | 17.8 | 0 | 0 |
| 3.5 | 0.17 | 54.8 | 22.0 | 23.2 | 0 | 0 |
| 5 | - | - | - | - | - | - |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

### Example 6: Results obtained in the epoxidation of olefinic compounds in the presence of O₂ with the catalyst described in Example 1 a [Au/CeO₂ - CAT A], using azo-iso-butyronitrile (AIBN) as initiator or activator.

Into a 12 ml stainless steel autoclave reactor, with an inner lining of Teflon and containing a magnetic stirrer, were placed 3000 mg of 1-octene, 1000 mg of 3-methyl-pentane and 12 mg of AIBN, followed by the addition of 85 mg of a catalyst as described in Example 1 a [Au/CeO₂ - CAT A]. The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 1.05 | 67.3 | 4.8 | 12.2 | 0 | 15.7 |
| 2 | 5.34 | 51.3 | 6.0 | 21.1 | 2.0 | 19.6 |
| 3.5 | 6.32 | 42.4 | 7.6 | 18.8 | 2.2 | 29.0 |
| 5 | 5.81 | 44.8 | 8.3 | 20.0 | 6.1 | 20.8 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

**Example 7**: Results obtained in the epoxidation of olefinic compounds in the presence of O₂ with the catalyst described in Example 2b [Ti-MCM-41-Sil. - CAT T], using azo-iso-butyronitrile (AIBN) as activator. Into a 12 ml stainless steel autoclave reactor, with an inner lining of Teflon and containing a magnetic stirrer, were placed 3000 mg of 1-octene, 1000 mg of 1-methyl-pentane and 12 mg of AIBN (initiator), followed by the addition of 100 mg of a catalyst as described in Example 2b [Ti-MCM-41-Sil. - CAT T] . The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide³ | Enol^{b} | Enone^{c} | Glycol^{d} | Dimers^{e} |
| 1 | 0.78 | 59.3 | 4.7 | 36.0 | 0 | 0 |
| 2 | 2.22 | 62.7 | 3.5 | 27.9 | 1.6 | 3.3 |
| 3.5 | 3.05 | 63.2 | 3.5 | 18.9 | 2.5 | 11.9 |
| 5 | 3.22 | 62.5 | 5.7 | 13.8 | 3.6 | 14.4 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

### Example 8: Results obtained in the epoxidation of olefinic compounds in the presence of 02 with the combination of catalysts described in Examples 1a and 2b [Au/CeO₂ - CAT A] +[Ti-MCM-41-Sil. - CAT T], using azo-iso-butyronitrile (AIBN) as initiator or activator.

Into a 12 ml stainless steel autoclave reactor, with an inner lining of Teflon and containing a bar magnet, were placed 3000 mg of 1-octene, 1000 mg of 3-methyl-pentane and 12 mg of AIBN (initiator), followed by the addition of a mechanical mixture of 50 mg of a catalyst as described in Example 1 a [Au/CeO₂ - CAT A] + 100 mg of a catalyst as described in Example 2b [Ti-MCM-41-Sil.CAT T].

The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds
(initial moles of reactant - final moles of reactant / initial moles of reactant * 100), and the selectivities of the products obtained (moles of product i / moles of total products *100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.80 | 92.8 | 3.5 | 3.7 | 0 | 0 |
| 2 | 1.85 | 92.4 | 3.4 | 2.4 | 1.4 | 0.4 |
| 3.5 | 2.98 | 88.5 | 3.4 | 2.4 | 3.6 | 2.1 |
| 5 | 4.79 | 87.4 | 2.7 | 2.4 | 5.1 | 2.4 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

### Example 9: Results obtained in the epoxidation of olefinic compounds in the presence of O₂ with the catalyst of Example 1b [Au/MCM-41 - CAT A], using azo-isobutyronitrile (AIBN) as initiator or activator.

Into a 12 ml stainless steel autoclave reactor, with an inner lining of Teflon and containing a magnetic stirrer, were placed 3000 mg of 1-octene, 1000 mg of 3-methyl-pentane and 12 mg of AIBN (initiator), followed by the addition of 85 mg of a catalyst as described in Example 1 b [Au/MCM-41 - CAT A]. The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds
(initial moles of reactant - final moles of reactant / initial moles of reactant * 100), and the selectivities of the products obtained (moles of product i / moles of total products *100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.21 | 11.5 | 11.5 | 8.5 | 12.4 | 18.7 |
| 2 | 0.63 | 11.2 | 18.9 | 7.4 | 10.1 | 26.6 |
| 3.5 | 1.24 | 10.0 | 27.4 | 21.3 | 5.1 | 39.2 |
| 5 | 1.49 | 15.6 | 26.7 | 25.6 | 4.8 | 40.3 |

| | | | | | | |
|---|---|---|---|---|---|---|
| a- Epoxide= 1*,*2-epoxy-octane. b- Enol = 1-octen-3-ol. c- Enone = 1-octen-3-one. d- 1,2-octanediol. Dimers = Products of dimerisation of olefinic compound. | | | | | | |

### Example 10: Results obtained in the epoxidation

Results obtained in the epoxidation of olefinic compounds in the presence of O₂ with the combination of catalysts described in Examples 1a and 2b [Au/MCM-41 - CAT A] + [Ti-MCM-41-Sil. - CAT T], using azo-iso-butyronitrile (AIBN) as initiator or activator.

Into a 12 ml stainless steel autoclave reactor, with an inner lining of Teflon and containing a bar magnet, were placed 3000 mg of 1-octene, 1000 mg of 3-methyl-pentane and 12 mg of AIBN (initiator), followed by the addition of a mechanical mixture of 50 mg of a catalyst as described in Example 1 b [Au/MCM-41 - CAT A] + 100 mg of a catalyst as described in Example 2b [Ti-MCM-41-Sil. - CAT T] . The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 1.13 | 58.4 | 5.9 | 9.3 | 7.3 | 19.1 |
| 2 | 1.58 | 45.6 | 8.9 | 8.2 | 10.7 | 26.6 |
| 3.5 | 1.72 | 45.0 | 10.0 | 10.1 | 10.9 | 23.9 |
| 5 | 2.17 | 43.2 | 15.0 | 11.9 | 10.8 | 19.3 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

### Example 11: Results obtained in the epoxidation of olefinic compounds in the presence of O₂ with cyclohexane as hydrocarbon and with the combination of catalysts of Examples 1 a and 2b [Au/CeO₂ - CAT A] + [Ti-MCM-41-Sil. - CAT T], using azo-iso-butyronitrile (AIBN) as initiator or activator.

Into a 12 ml stainless steel autoclave reactor, with an inner lining of Teflon and containing a bar magnet, were placed 3000 mg of 1-octene, 1000 mg of cyclohexane and 12 mg of AIBN (initiator), followed by the addition of a mechanical mixture of 50 mg of a catalyst as described in Example 1a [Au/CeO₂ - CAT A] + 100 mg of a catalyst as described in Example 2b [Ti-MCM-41-Sil. - CAT T]. The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.47 | 90.3 | 5.7 | 4.0 | 0 | 0 |
| 2 | 2.10 | 85.2 | 4.7 | 5.6 | 3.9 | 0.6 |
| 3.5 | 2.90 | 82.3 | 5.7 | 6.8 | 3.7 | 1.5 |
| 5 | 4.10 | 83.0 | 6.4 | 6.5 | 2.1 | 2.0 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

### Example 12: Results obtained in the epoxidation of olefinic compounds in the presence of O₂ with ethyl-benzene as hydrocarbon and with the combination of catalysts of Examples 1a and 2b [Au/CeO₂ - CAT A] + [Ti-MCM-41-Sil. - CAT T], using azo-iso-butyronitrile (AIBN) as initiator or activator.

Into a 12 ml stainless steel autoclave reactor, with an inner lining of Teflon and containing a bar magnet, were placed 3000 mg of 1-octene, 1250 mg of ethyl-benzene and 12 mg of AIBN (initiator), followed by the addition of a mechanical mixture of 50 mg of a catalyst as described in Example 1a [Au/CeO₂ - CAT A] + 100 mg of a catalyst as described in Example 2b [Ti-MCM-41-Sil. - CAT T]. The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant *100), and the selectivities of the products obtained (moles of product i / moles of total products *100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.64 | 88.3 | 3.1 | 4.6 | 4.0 | 0 |
| 2 | 1.98 | 86.7 | 2.6 | 4.7 | 3.8 | 2.2 |
| 3.5 | 3.44 | 85.0 | 3.6 | 4.0 | 5.3 | 2.1 |
| 5 | 4.89 | 86.1 | 3.6 | 4.2 | 4.3 | 1.8 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

### Example 13: Results obtained in the epoxidation of olefinic compounds in the presence of O₂ with iso-propyl-benzene (cumene) as hydrocarbon and with the combination of catalysts of Examples 1 a and 2b [Au/CeO₂ - CAT A] + [Ti-MCM-41-Sil. - CAT T], using azo-iso-butyronitrile (AIBN) as initiator or activator.

Into a 12 ml stainless steel autoclave reactor, with an inner lining of Teflon and containing a bar magnet, were placed 3000 mg of 1-octene, 1360 mg of iso-propyl-benzene (cumene) and 12 mg of AIBN (initiator), followed by the addition of a mechanical mixture of 50 mg of a catalyst as described in Example 1 a [Au/CeO₂ - CAT A] + 100 mg of a catalyst as described in Example 2b [Ti-MCM-41-Sil. - CAT T]. The autoclave is hermetically sealed, the lid having a connection to a pressure gauge (manometer), with another connection to load the source of gaseous oxygen and a third outlet allowing samples to be taken at different time intervals. The reactor is pressurised with oxygen at 10 bars, and the reaction temperature raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products * 100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enone^{c} | Glycol^{d} | Dimers^{e} |
| 1 | 0.97 | 94.5 | 3.0 | 2.5 | 0 | 0 |
| 2 | 1.52 | 93.5 | 2.9 | 2.0 | 1.6 | 0 |
| 3.5 | 3.27 | 92.6 | 3.0 | 1.9 | 1.1 | 1.4 |
| 5 | 5.33 | 91.7 | 3.1 | 2.8 | 1.3 | 1.1 |

| | | | | | | |
|---|---|---|---|---|---|---|
| a- Epoxide= 1,2-epoxy-octane- b- Enol = 1-octen-3-ol. c- Enone = 1-octen-3-one. d- 1,2-octanediol. Dimers = Products of dimerisation of olefinic compound. | | | | | | |

### Example 14: Results obtained in the epoxidation of olefinic compounds with a constant and controlled feed of O₂, with iso-propyl-benzene (cumene) as the hydrocarbon and with the combination of catalysts of Examples 1a and 2b [Au/CeO₂ - CAT A] + [Ti-MCM-41-Sil. - CAT T], using azo-iso-butyronitrile (AIBN) as initiator or activator.

Into a 12 ml stainless steel autoclave reactor, with an inner lining of Teflon and containing a magnetic stirrer, were placed 3000 mg of 1-octene and 1360 mg of iso-propyl-benzene (cumene) and 12 mg of AIBN (initiator), followed by the addition of a mechanical mixture of 50 mg of a catalyst as described in Example 1 a [Au/CeO₂ - CAT A] + 100 mg of a catalyst as described in Example 2b [Ti-MCM-41-Sil. - CAT T] . The autoclave is hermetically sealed, the lid having a connection to a pressure gauge (manometer), with another connection to load the source of gaseous oxygen, with a 10-bar pressure regulator, and a third outlet allowing samples to be taken at different time intervals. The reactor is pressurised at 10 bars with oxygen and this pressure is maintained constant throughout the process through the slow addition of oxygen into the system. The reaction temperature is raised to 90 °C, and the autoclave immersed in a silicone bath with temperature control. The reaction mixture is shaken and samples taken at various time intervals up to a reaction time of 17 hours. The samples are analysed using GC with an FID detector, to calculate the composition of the mixture obtained, the conversion of olefinic compounds (initial moles of reactant - final moles of reactant / initial moles of reactant * 100), and the selectivities of the products obtained (moles of product i / moles of total products *100) in each case. The following results were obtained in this manner:

| Time (hours) | Conversion (% Mol.) | Selectivity (% Mol.) | | | | |
|---|---|---|---|---|---|---|
| | | Epoxide^{a} | Enol^{b} | Enona^{c} | Glycol^{d} | Dimers^{e} |
| 3.5 | 3.33 | 92.4 | 3.1 | 2.1 | 1.2 | 1.2 |
| 7.0 | 6.52 | 91.5 | 3.1 | 2.8 | 1.4 | 1.2 |
| 10.5 | 8.74 | 89.9 | 3.5 | 3.5 | 1.6 | 1.8 |

| | | | | | | |
|---|---|---|---|---|---|---|
| a- Epoxide = 1,2-epoxy-octane. b- Enol = 1-octen-3-ol. c- Enone = 1-octen-3-one. d- 1,2-octanediol. Dimers = Products of dimerisation of olefinic compound. | | | | | | |

## Claims

1. Procedure for the epoxidation of olefinic compounds, **characterised in that** it comprises performing an oxidation reaction of at least one olefinic compound containing one or more double C=C bonds, with oxygen, in the presence of:
- one or more reaction initiator agents, and
- at least one catalyst, which comprises at least one metal selected from among one or more noble metals, one or more transition metals and mixtures thereof, and
- at least one hydrocarbon selected from among one or more branched alkanes, one or more cyclic or alkyl-cyclic hydrocarbons, one or more aromatic or alkyl-aromatic compounds, and mixtures thereof.

2. Procedure according to claim 1, **characterised in that** an organic epoxide is obtained, this being an olefin selected from among those which are terminal, internal, linear, cyclic, unbranched, or with one or more chain branches, and comprises a hydrocarbon chain of between 2 and 24 carbon atoms.

3. Procedure according to any of claims 1 or 2, **characterised in that** an organic epoxide is obtained having the general formula:
R₁-HCOCH-R₂
Where R₁ and R₂ are the same or different replacements, selected freely from among: alkyl with from 1 to 12 C atoms, linear or branched, substituted or unsubstituted; cyclic alkyl with from 4 to 12 C atoms, substituted or unsubstituted o aryl with from 6 to 18 C atoms, substituted or unsubstituted.

4. Procedure according to any of claims 1 or 2, **characterised in that** an organic epoxide is obtained with the formula:
R₁-HCOCH- (CH₂) ₙ-HCOCH-R₂
where R₁ and R₂ are the same or different replacements, selected freely from among: alkyl with from 1 to 12 C atoms, linear or branched, substituted or unsubstituted; cyclic alkyl with from 4 to 12 C atoms, substituted or unsubstituted; or aryl with from 6 to 18 C atoms, substituted or unsubstituted; and n may vary between 1 and 12.

5. Procedure according to any of claims 1 or 2, **characterised in that** an organic epoxide is obtained having between 2 and 12 carbon atoms.

6. Procedure according to claim 1, **characterised in that** the olefinic compound is selected from among a mono-olefin, a di-olefin and a polyolefin.

7. Procedure according to claim 1, **characterised in that** the olefinic compound is a mono-olefin.

8. Procedure according to claim 7, **characterised in that** the said mono-olefin is selected from among one or more terminal, internal, branched, cyclic olefins, and combinations thereof.

9. Procedure according to claim 1, **characterised in that** the hydrocarbon is a compound corresponding to the formula:
R₁-(CH₂)ₙ-R₂
where R₁ and R₂ are the same or different replacements, selected freely from among: hydrogen, branched alkyl with from 1 to 12 C atoms, substituted or unsubstituted; cyclic alkyl with from 4 to 12 C atoms, substituted or unsubstituted; or aryl with from 6 to 18 C atoms, substituted or unsubstituted; and n may vary between 0 and 12.

10. Procedure according to claim 1, **characterised in that** the olefinic compound is propylene and the hydrocarbon is a methyl-alkane or mixtures of methyl-alkanes.

11. Procedure according to claim 1, **characterised in that** the olefinic compound is propylene and the hydrocarbon is ethyl-benzene.

12. Procedure according to claim 1, **characterised in that** the olefinic compound is propylene and the hydrocarbon is iso-propyl-benzene (cumene).

13. Procedure according to claim 1, **characterised in that** the reaction initiator agent is selected from among:
- -one or more organic nitriles,
- one or more azo-compounds,
- mixtures of one or more organic nitriles and one or more azo-compounds, and
- one or more organic nitriles comprising one or more azo- groups in the same molecule.

14. Procedure according to claim 13, **characterised in that** the initiator agent is an organic compound with at least one nitrile group in its composition, and at least one azo- group in the same molecule, and corresponding to the general formula:
N≡C-R₁-N=N-R₂-C≡N
where R₁ and R₂ are the same or different replacements, selected freely from among: an alkyl group of from 1 to 12 C atoms, linear or branched, substituted or unsubstituted; an alkyl-cyclic group of from 4 to 12 C atoms, substituted or unsubstituted; and an aryl group of from 6 to 18 C atoms, substituted or unsubstituted.

15. Procedure according to claim 1, **characterised in that** the oxygen is derived from a source selected from among molecular oxygen, a gaseous mixture comprising oxygen and combinations thereof.

16. Procedure according to claim 15, **characterised in that** the source of oxygen is a gaseous mixture including oxygen, selected from among air, oxygen-enriched air, oxygen-enriched ozone, oxygen-enriched N₂, oxygen-enriched Ar, and a mixture comprising nitrogen, argon and oxygen.

17. Procedure according to claim 1, **characterised in that** the catalyst is selected from among:
a) a metallic catalyst *"CAT A"* comprising
- one or more noble metals, or
- one or more transition metals, or
- one or more of their salts or complexes, and
- combinations of the above,
with the aforementioned *"CAT A"* being supported on, or included within, the structure of an inorganic matrix;
b) a metallic catalyst "CAT T" comprising one or more transition metals, their salts or complexes, included within or supported on the structure of an inorganic matrix; and
c) combinations thereof *"CAT A"* + *"CAT T*".

18. Procedure according to claim 17, **characterised in that** the catalyst is a metallic catalyst "CAT A" , comprising one or more noble metals, one or more transition metals, or one of their salts, and is supported on, or included within, the structure of an inorganic matrix.

19. Procedure according to claim 17, **characterised in that** the catalyst is a metallic catalyst "CAT T", comprising one or more transition metals, included within the structure of an inorganic matrix.

20. Procedure according to claim 18, **characterised in that** the said noble metal is selected from among Au, Pd, Pt, Ag, Re, Rh, and combinations thereof.

21. Procedure according to claim 18, **characterised in that** the said noble metal is Au or Au combined with another metal.

22. Procedure according to claim 21, **characterised in that** the said Au, or Au combined with another metal, is supported in the form of nano-particles of a size of between 0.5 and 20 nm.

23. Procedure according to any of claims 17, 18 or 19, **characterised in that** the said transition metal is selected from among one or more metals of groups lb, llb, lVb, Vb, Vlb, Vllb and VIII of the periodic table.

24. Procedure according to any of claims 17, 18 or 19, **characterised in that** the said transition metal is selected from among Ti, Zr, Zn, Cu, Co, Mn, Mo, V, Ni, Fe, Al, and combinations thereof.

25. Procedure according to claim 18, **characterised in that** the said inorganic matrix is an amorphous material selected from among one or more metal oxides, one or more mixed metal oxides, and combinations thereof.

26. Procedure according to claim 25, **characterised in that** the said inorganic matrix is selected from among: silica, alumina, ceria, yttria, titania, Fe₂O3, silica-alumina, silica-ceria, one or more mixed alkaline earth metal oxides, and one or more transition metal oxides.

27. Procedure according to claim 18, **characterised in that** the said inorganic matrix is an oxide of cerium.

28. Procedure according to any of claims 17, 18 or 19, **characterised in that** the said inorganic matrix is one or more micro-porous molecular sieves.

29. Procedure according to 28, **characterised in that** the said micro-porous molecular sieve is selected from among a zeolite, clay and pillared clay, and mixtures thereof.

30. Procedure according to any of claims 17, 18 or 19, **characterised in that** the said inorganic matrix is one or more meso-porous molecular sieves.

31. Procedure according to in claim 30, **characterised in that** the said meso-porous molecular sieve is selected from among silicate, metal-silicate and a meso-porous material derived from the delamination of a laminar zeolitic precursor.

32. Procedure according to claim 19, **characterised in that** the said inorganic matrix is an amorphous material selected from among silica, alumina, silica-alumina, titania, silica-titania, and a mixed transition metal oxide.

33. Procedure according to claim 30, **characterised in that** the said meso-porous solid is selected from among meso-porous molecular sieves, and molecular sieves containing meso- and micro-pores, and **in that** it contains at least Si, Ti incorporated within the grid, in tetrahedral positions.

34. Procedure according to claim 33, **characterised in that** the said meso-porous solid comprises in addition Ti in non-reticular (octahedral) positions of the molecular sieve, and silicon bound to carbon.

35. Procedure according to claim 30, **characterised in that** the meso-porous molecular sieve corresponds in its calcined and anhydrous form, without an organic component, to the chemical composition
y *(A*_{*1*/}*ₙⁿ⁺* XO₂) : t TO₂ : SiO₂ : x TiO₂ where:
X represents at least one trivalent element,
- y lies between 0 and 0.2,
- A represents mono-, di- or tri-valent compensation cations, or mixtures of these, - n = 1, 2 or 3,
- T represents at least one tetravalent element other than Si and Ti,
- t lies between 0 and 1, and
- x may range from 0.015 to 0.065.

36. A procedure according to claim 30, **characterised in that** the solid meso-porous catalyst is selected from among materials of the type MCM-41, MCM-48, SBA-15, HMS and mixtures thereof.

37. A procedure according to claim 30, **characterised in that** the solid meso-porous catalyst has been prepared by means of a process comprising a stage selected from among synthesis and post-synthesis stages, during which Si-C bonds are introduced into the catalyst.

38. Procedure according to claim 1, **characterised in that** the epoxidation of olefinic compounds takes place in a reactor selected from among a discontinuous reactor, a CSTR reactor, a continuous fixed bed reactor, a fluidised bed reactor and an ebullient bed reactor.

39. Procedure according to claim 1, **characterised in that** the epoxidation of olefinic compounds takes place within a discontinuous reactor, with the weight proportion of the olefinic compound to the catalyst lying within the range between 2 and 1000.

40. Procedure according to claim 39, **characterised in that** the said weight proportion of the olefinic compound to the catalyst lies within the range between 10 and 500.

41. Procedure according to claim 1, **characterised in that** the epoxidation of the olefinic compound takes place within a discontinuous reactor, with a molar proportion of the olefinic compound and the oxidant within the range between 3 and 600.

42. Procedure according to claim 1, **characterised in that** the epoxidation of olefinic compounds takes place within a discontinuous reactor, with a weight proportion of olefinic compound and initiator agent lying within the range between 10 and 10000.

43. Procedure according to claim 1, **characterised in that** the epoxidation reaction of olefinic compounds takes place within a discontinuous reactor, with a weight proportion of olefinic compound and the hydrocarbon lying within the range between 0.1 and 200.

44. Procedure according to claim 1, **characterised in that** the epoxidation of olefinic compounds takes place within a discontinuous reactor, at a temperature of between 10 and 250°C.

45. Procedure according to claim 44, **characterised in that** the temperature lies between 40 and 200°C.

46. Procedure according to claim 1, **characterised in that** the epoxidation reaction of olefinic compounds takes place within a discontinuous reactor, over a reaction time of between 2 minutes and 72 hours.

47. Procedure according to claim 1, **characterised in that** the epoxidation of olefinic compounds takes place within a discontinuous reactor, at a total pressure within the system of between atmospheric pressure and 50 bars.

48. Procedure according to claim 1, **characterised in that** the epoxidation of olefinic compounds takes place within a discontinuous reactor, with the continuous addition of oxygen, maintaining the pressure of the reactor constant, at a total pressure within the system of between atmospheric pressure and 50 bars.
